(19) Europäisches Patentamt
European Patent Office
Office européen des brevets

(11) **EP 4 503 045 A1**

(12) **EUROPEAN PATENT APPLICATION**
published in accordance with Art. 153(4) EPC

(43) Date of publication:
**05.02.2025 Bulletin 2025/06**

(21) Application number: **23779036.5**

(22) Date of filing: **20.02.2023**

(51) International Patent Classification (IPC):
*G16C 60/00* (2019.01)    *G01N 23/2251* (2018.01)
*G01N 33/00* (2006.01)    *G06T 7/00* (2017.01)

(52) Cooperative Patent Classification (CPC):
**G01N 23/2251; G01N 33/00; G06T 7/00;
G06V 10/776; G16C 60/00**

(86) International application number:
**PCT/JP2023/006045**

(87) International publication number:
**WO 2023/189006 (05.10.2023 Gazette 2023/40)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **30.03.2022 JP 2022055035**

(71) Applicants:
• **OSAKA UNIVERSITY
Suita-shi
Osaka 565-0871 (JP)**
• **GC Corporation
Sunto-gun, Shizuoka 410-1307 (JP)**

(72) Inventors:
• **YAMAGUCHI, Satoshi
Suita-shi, Osaka 565-0871 (JP)**
• **IMAZATO, Satoshi
Suita-shi, Osaka 565-0871 (JP)**
• **HOKII, Yusuke
Tokyo 174-8585 (JP)**
• **AKIYAMA, Shigenori
Tokyo 174-8585 (JP)**
• **FUSEJIMA, Futoshi
Tokyo 174-8585 (JP)**

(74) Representative: **Müller-Boré & Partner
Patentanwälte PartG mbB
Friedenheimer Brücke 21
80639 München (DE)**

(54) **MATERIAL PROPERTY VALUE PREDICTION METHOD, TRAINED MODEL GENERATION METHOD, PROGRAM, AND DEVICE**

(57) An accuracy of prediction of a characteristic value of a material is improved. A method according to one aspect of the present invention includes acquiring an image of a material, performing a topological data analysis on the image of the material to extract features of the material, and predicting a characteristic value of the material from the features of the material.

FIG.2

**Description**

TECHNICAL FIELD

The present invention relates to a method of predicting a characteristic value of a material, a method of generating a trained model, programs, and devices.

BACKGROUND ART

In material development, an approach that has been taken is such that a material is actually produced and then characteristic values of the produced material are evaluated. Currently, a method of predicting a characteristic value of a material using machine learning, so-called Materials Informatics, is also used.

Citation List

Patent Document

Patent Document 1: Japanese Unexamined Patent Application Publication No. 2021-111360

SUMMARY OF THE INVENTION

Technical Problem

However, there is a need in material development for predicting a characteristic value of a material with higher accuracy. An object of the present invention is to improve accuracy of prediction of a characteristic value of a material.

Solution to Problem

A method according to one embodiment of the present invention includes acquiring an image of a material, performing a topological data analysis on the image of the material to extract features of the material, and predicting a characteristic value of the material from the features of the material.

Effects of the Invention

The present invention can improve accuracy of prediction of a characteristic value of a material.

BRIEF DESCRIPTION OF THE DRAWINGS

[Fig. 1] Fig. 1 is a diagram illustrating an overall configuration according to one embodiment of the present invention.
[Fig. 2] Fig. 2 is a functional block diagram of a prediction device according to one embodiment of the present invention.
[Fig. 3] Fig. 3 is a functional block diagram of a learning device according to one embodiment of the present invention.
[Fig. 4] Fig. 4 is a flowchart of a prediction process according to one embodiment of the present invention.
[Fig. 5] Fig. 5 is a flowchart of a learning process according to one embodiment of the present invention.
[Fig. 6] Fig. 6 is a diagram for explaining division of an image according to one embodiment of the present invention.
[Fig. 7] Fig. 7 is a diagram for explaining preprocessing of an image according to one embodiment of the present invention.
[Fig. 8] Fig. 8 is a diagram for explaining a topological data analysis (persistent homology) according to one embodiment of the present invention.
[Fig. 9] Fig. 9 is a diagram for explaining dimensionality reduction of a vector according to one embodiment of the present invention.
[Fig. 10] Fig. 10 is a diagram for explaining dimensionality reduction of a vector according to one embodiment of the present invention.
[Fig. 11] Fig. 11 is a diagram for explaining machine learning according to one embodiment of the present invention.
[Fig. 12] Fig. 12 is a diagram for explaining an inverse analysis according to one embodiment of the present invention.
[Fig. 13] Fig. 13 is a diagram for explaining an inverse analysis according to one embodiment of the present invention.

[Fig. 14] Fig. 14 is a hardware configuration diagram of a prediction device and a learning device according to one embodiment of the present invention.

DETAILED DESCRIPTION OF THE INVENTION

Embodiments of the present invention will be described with reference to drawings hereinafter.

<Explanation of terminologies >

- In the present specification, a "material" may be any material. For example, the "material" is a medical material (e.g., a dental material). For example, the "material" is a ceramic, a glass-ceramic, a polymer material, a composite resin, a glass ionomer, or a metal (e.g., a dental ceramic, a dental glass-ceramic, a dental polymer material, a dental composite resin, a dental glass ionomer, and a dental metal).
- In the present specification, a "characteristic value" may be any characteristic value. For example, the "characteristic value" is a mechanical property (e.g., a biaxial flexural strength, abrasion resistance, and the like).

<Overall configuration>

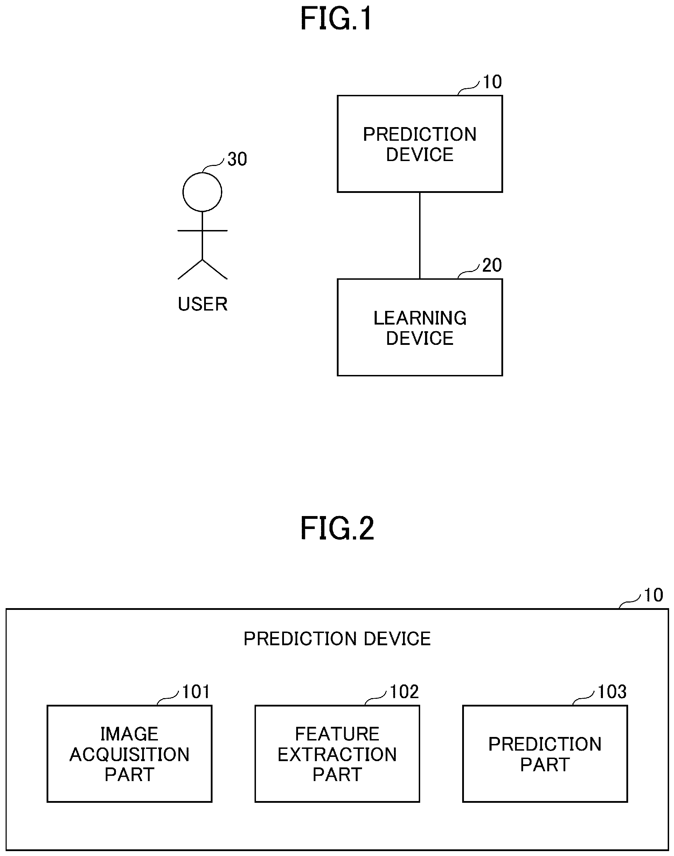

Fig. 1 is a diagram illustrating an overall configuration according to one embodiment of the present invention. A user 30 operates a prediction device 10 and a learning device 20. Although the prediction device 10 and the learning device 20 are explained as separate devices in Fig. 1, the prediction device 10 and the learning device 20 may be implemented as a single device.

<<Prediction device»

The prediction device 10 is a device configured to predict a characteristic value of a material. The prediction device 10 is composed of one computer or multiple computers. The prediction device 10 can transmit and receive data to and from the learning device 20 via an arbitrary network.

<<Learning device»

The learning device 20 is a device configured to generate a trained model used for predicting a characteristic value of a material. The learning device 20 is composed of one computer or multiple computers. The learning device 20 can transmit and receive data to and from the prediction device 10 via an arbitrary network.

functional blocks>

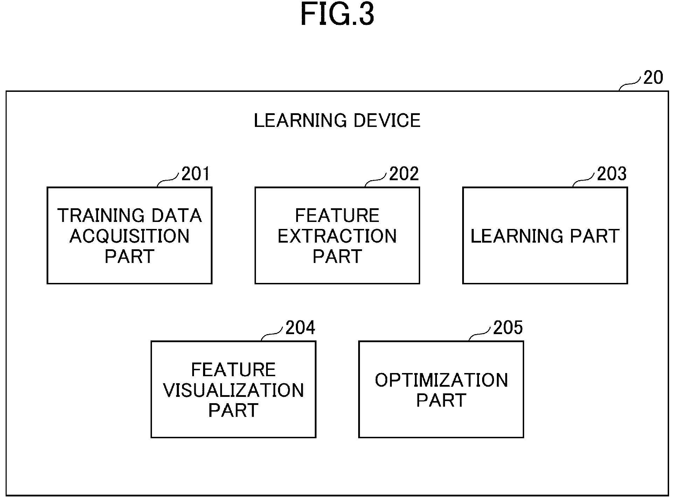

Hereinafter, functional blocks of the prediction device 10 will be explained with reference to Fig. 2, and functional blocks of the learning device 20 will be explained with reference to Fig. 3.

Fig. 2 is a functional block diagram of the prediction device 10 according to one embodiment of the present invention. The prediction device 10 includes an image acquisition part 101, a feature extraction part 102, and a prediction part 103. As a program is executed, the prediction device 10 functions as the image acquisition part 101, the feature extraction part 102, and the prediction part 103.

The image acquisition part (may be merely referred to as an acquisition part) 101 is configured to acquire an image of a material. Note that, the image acquisition part 101 may divide the acquired image and use the divided image. For example, the image is a scanning electron microscopic (SEM) image.

The feature extraction part 102 performs a topological data analysis on the image of the material (the divided image) acquired by the image acquisition part 101 to extract features of the material. For example, the topological data analysis is persistent homology. Note that, the feature extraction part 102 may perform dimensionality reduction (e.g., a principal component analysis) of the extracted features of the material.

The prediction part 103 predicts a characteristic value of the material from the features of the material using a trained model generated by the learning device 20.

Fig. 3 is a functional block diagram of the learning device 20 according to one embodiment of the present invention. The learning device 20 includes a training data acquisition part 201, a feature extraction part 202, a learning part 203, a feature visualization part 204, and an optimization part 205. As a program is executed, the learning device 20 functions as the training data acquisition part 201, the feature extraction part 202, a learning part 203, a feature visualization part 204, and an optimization part 205.

The training data acquisition part (may be merely referred to as an acquisition part) 201 acquires training data used for generating a trained model. Specifically, the training data acquisition part 201 acquires an image of a material, and an actual measurement value of a characteristic value of the material. Note that, the training data acquisition part 201 may divide the acquired image, and use the divided image. For example, the image is a scanning electron microscopic (SEM) image.

The feature extraction part 202 performs a topological data analysis on the image of the material (or the divided image) acquired by the training data acquisition part 201 to extract features of the material. For example, the topological data analysis is persistent homology. Note that, the feature extraction part 202 may perform dimensionality reduction (e.g., a principal component analysis) of the extracted features of the material.

The learning part 203 produces a machine learning model with the features of the material and the actual measurement value of the characteristic value of the material to generate a trained model for predicting a characteristic value of the material from the features of the material.

The feature visualization part 204 visualizes the features of the material.

The optimization part 205 determines parameters for extracting the features of the material through Bayesian optimization.

<Processing method>

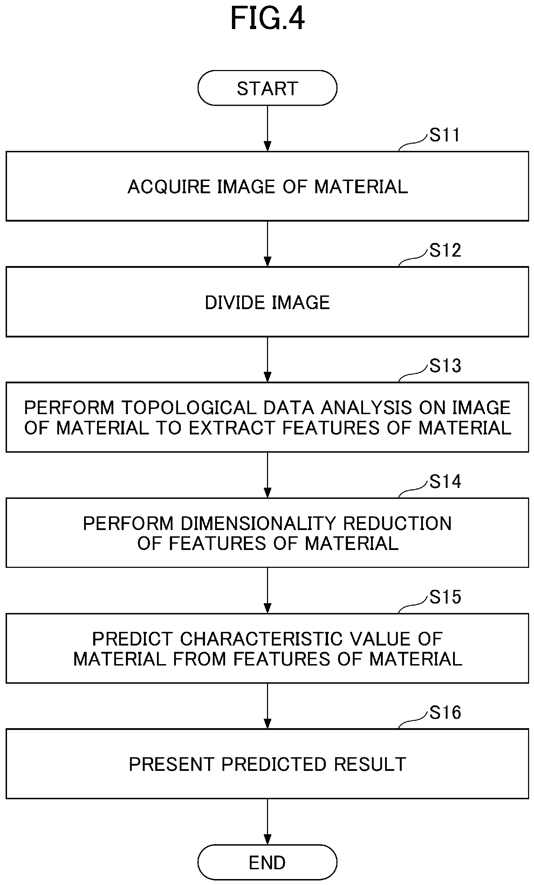

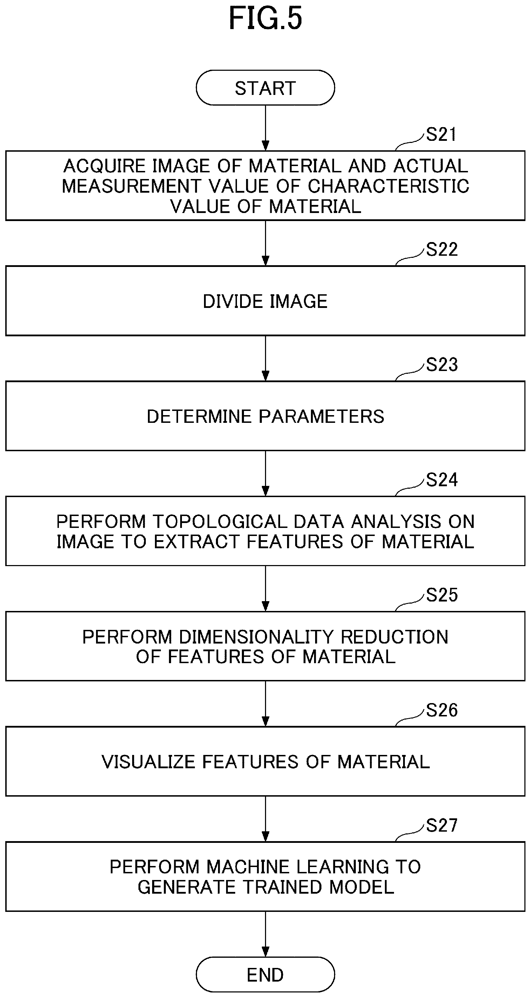

Hereinafter, a prediction process will be explained with reference to Fig. 4, and a learning process will be explained with reference to Fig. 5.

Fig. 4 is a flowchart of a prediction process according to one embodiment of the present invention.

In step 11 (S11), the image acquisition part 101 of the prediction device 10 acquires an image of a material.

In step 12 (S12), the image acquisition part 101 of the prediction device 10 divides the image acquired in S11. Note that, S12 can be omitted.

In step 13 (S13), the feature extraction part 102 of the prediction device 10 performs a topological data analysis on the image of the material acquired in S11 or the image divided in S12 to extract features of the material.

In step 14 (S14), the feature extraction part 102 of the prediction device 10 performs dimensionality reduction (e.g., a principal component analysis) of the features of the material extracted in S13. Note that, S14 can be omitted.

In step 15 (S15), the prediction part 103 of the prediction device 10 predicts a characteristic value of the material from the features of the material extracted in S13 or the features of the material subjected to dimensionality reduction in S14 using the trained model generated by the learning device 20.

In step 16 (S16), the prediction part 103 of the prediction device 10 presents (e.g., by displaying on a screen) the predicted result of S15 to the user 30.

Fig. 5 is a flowchart of the learning process according to one embodiment of the present invention.

In step 21 (S21), the training data acquisition part 201 of the learning device 20 acquires training data used for generating a trained model. Specifically, the training data acquisition part 201 acquires an image of a material, and an actual measurement value of a characteristic value of the material.

In step 22 (S22), the training data acquisition part 201 of the learning device 20 divides the image acquired in S21. Note that, S22 can be omitted.

In step 23 (S23), the optimization part 205 of the learning device 20 determines parameters used for extracting features of the material. For example, the optimization part 205 of the learning device 20 determines parameters used for extracting features of the material through Bayesian optimization.

In step 24 (S24), the feature extraction part 202 of the learning device 20 performs a topological data analysis on the image of the material acquired in S21 or the image divided in S22 to extract features of the material.

In step 25 (S25), the feature extraction part 202 of the learning device 20 performs dimensionality reduction (e.g., a principal component analysis) of the features of the material extracted in S24. Note that, S25 can be omitted.

In step 26 (S26), the feature visualization part 204 of the learning device 20 visualizes the features of the material.

In step 27 (S27), the learning part 203 of the learning device 20 learns the features of the material and the actual measurement value of the characteristic value of the material through machine learning to generate a trained model for predicting a characteristic value of the material from the features of the material.

Each process will be explained in detail hereinafter. As an example, a case where a dental glass-ceramic is used (note that the glass-ceramic is a glass-ceramic on which alkali etching is performed (i.e., a glass component of the glass-ceramic is dissolved) to expose crystal grains) will be explained.

<<Division of image>>

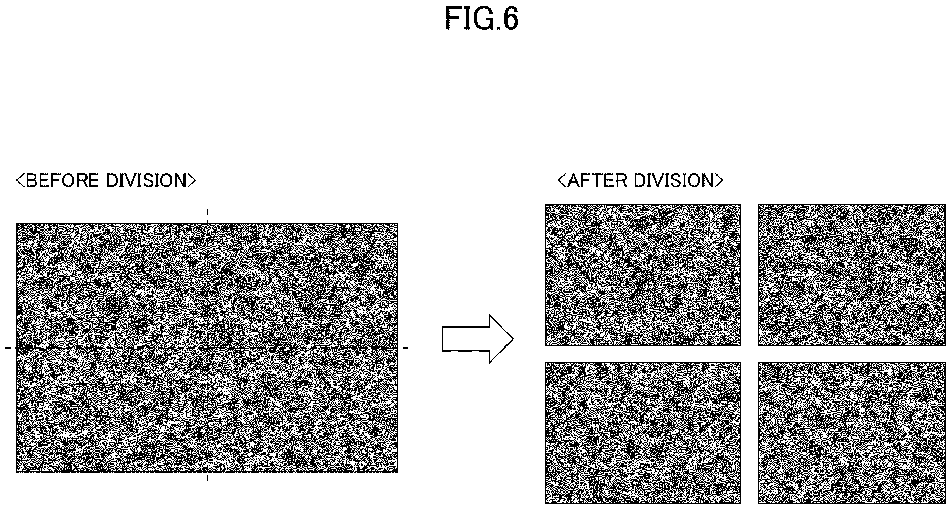

First, the prediction device 10 and the learning device 20 divide an SEM image. Fig. 6 is a diagram for explaining division of an image according to one embodiment of the present invention. The left side of Fig. 6 depicts the SEM image before the division, and the right side of Fig. 6 depicts the SEM image after the division.

In a case where an unnecessary portion is included in the SEM image, the unnecessary portion is cut out as depicted in <BEFORE DIVISION> on the left side of Fig. 6. Then, the SEM image is divided (divided into four in the example of Fig. 6).

As depicted in <AFTER DIVISION> on the right side of Fig. 6, the single SEM image is divided into two or more images. If the image is excessively divided, information included in the original image may be lost, which may lower the accuracy of the prediction. Therefore, the image is preferably divided into two to four. By dividing the image in the above-described manner, training data for machine learning can be increased. As the image is divided, moreover, when there is an uneven portion in an image of one material, the uneven portion can be extracted by a principal component analysis.

<<Preprocessing of image>>

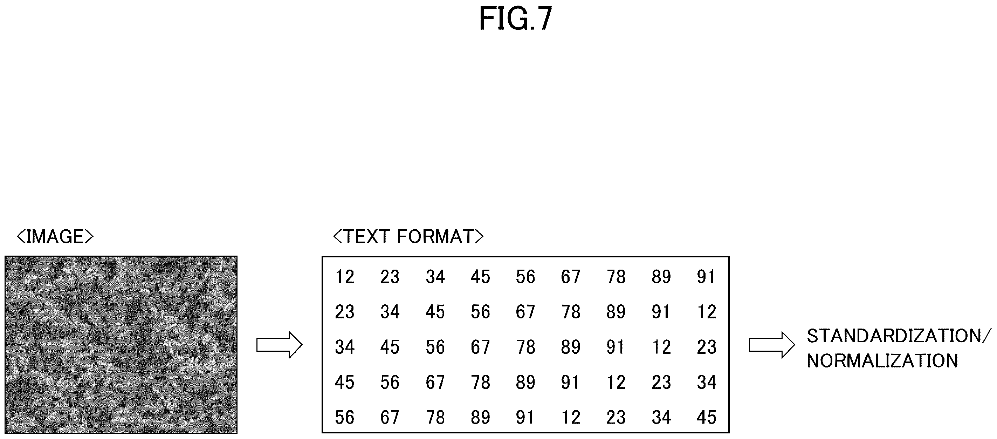

Next, the prediction device 10 and the learning device 20 perform preprocessing of the image. Fig. 7 is a diagram for explaining preprocessing of the image according to one embodiment of the present invention.

After unifying the gray scale of the image in 8 bits, the image is converted into a text format so that the image can be easily handled by a computer. One text-format file is produced for one image, and the files are grouped for each prototype or each product.

Since brightness of images may vary depending on a day on which an image is captured, a prototype, or a product, images are standardized or normalized. A calculation for standardization (scaling so that the average value becomes 0 and dispersion (standard deviation) becomes 1) or normalization (scaling so that the minimum value becomes 0 and the maximum value becomes 1) is performed on all the images using the average value, the maximum value, and the minimum value of the brightness of all of the images.

<<Topological data analysis (persistent homology) »

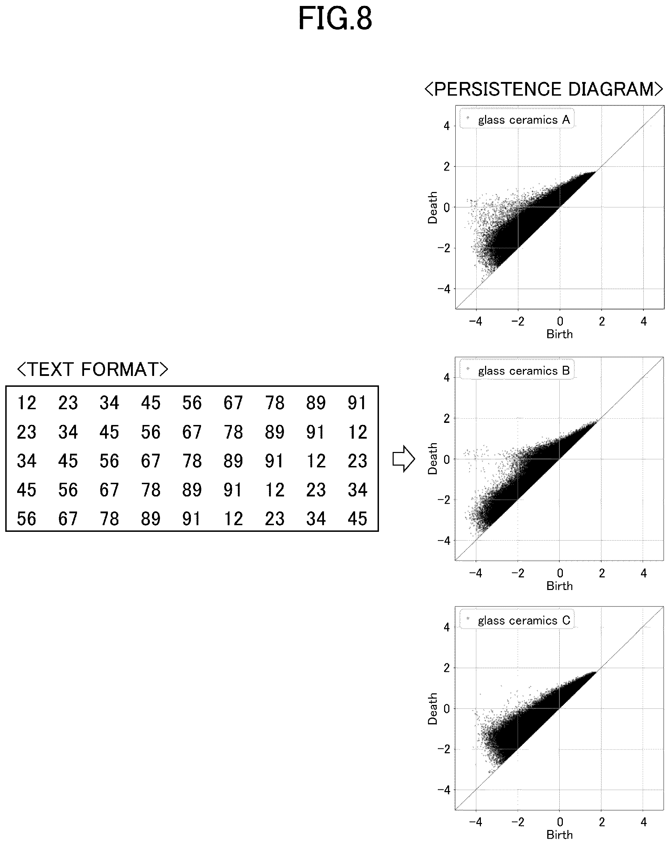

Next, the prediction device 10 and the learning device 20 performs a topological data analysis (persistent homology) on the image. Fig. 8 is a diagram for explaining the topological data analysis (persistent homology) according to one embodiment of the present invention.

In one embodiment of the present invention, a calculation of persistent homology is performed on each SEM image (specifically, a text format) to obtain n-dimensional persistence diagrams (e.g., a 0-dimensional persistence diagram and a 1-dimensional persistence diagram). Depending on an image that will be a subject of the analysis, the image is binarized in advance, and the binarized image may be subjected to a topological data analysis.

The persistent homology will be explained. The persistent homology is one of data analysis methods (topological data analysis) using a mathematical concept of topology, and quantitively represents a shape of data based on a structure of a shape, such as a connected component, ring, void, or the like of a shape. The persistence diagram represents an appearance (birth) and disappearance (death) of a connected component, ring, void, or the like of a shape. The 0-dimensional persistent homology computes a linkage between a point and another point, and the 1-dimensional persistent homology computes a relationship of a ring composed of a cluster of points. As in the above, use of persistent homology can reveal the topological features of the image of the material.

<<Extraction of features (vectorization)>>

Next, the prediction device 10 and the learning device 20 extract (vectorize) features from the persistence diagrams. Specifically, a method of persistence images (PI) (e.g., "Persistent Homology and Its Applications to Materials Science (https://www.jim.or.jp/journal/m/pdf3/58/01/17.pdf)") is used. The persistence diagram is divided into a grid (e.g., $128 \times 128$ sections), and the frequency (density) of the data points per section of the grid is determined as each element of a vector. It is determined that the frequency (density) conforms to a normal distribution.

The distribution function $\rho$ is represented by an equation (1). $D_k(X)$ is a k-dimensional persistence diagram of X, b is birth (i.e., appearance of a connected component, ring, void, or the like of a shape) and d is death (i.e., disappearance of the connected component, ring, void, or the like of the shape).

In accordance with an equation (2), a numerical value is weighed (using an arctangent function) according to a distance from a diagonal line on the persistence diagram. In this manner, an importance of each point on the persistence diagram can be reflected (the importance increases as the point is further from the diagonal line of the persistence diagram).

The $\sigma$ (standard deviation), C, and p are parameters, which need to be preset by a human. As described later, the parameters ($\sigma$ (standard deviation), C, and p) used for extracting features of a material can be determined by Bayesian optimization.

[Math. 1]

$$\rho(x,y) = \sum_{(b_i,d_i)\in D_k(X)} w(b_i,d_i)exp\left(-\frac{(x-b_i)^2+(y-d_i)^2}{2\sigma}\right)$$

$$\cdots\text{EQUATION (1)}$$

[Math. 2]

$$w(b,d) = arctan(C(d-b)^p) \qquad \cdots\text{EQUATION (2)}$$

<<Dimensionality reduction of vector>>

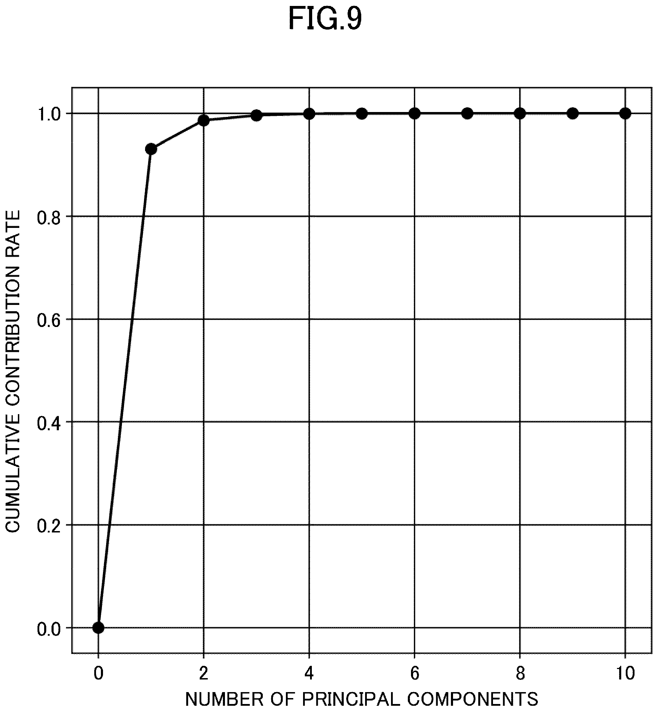

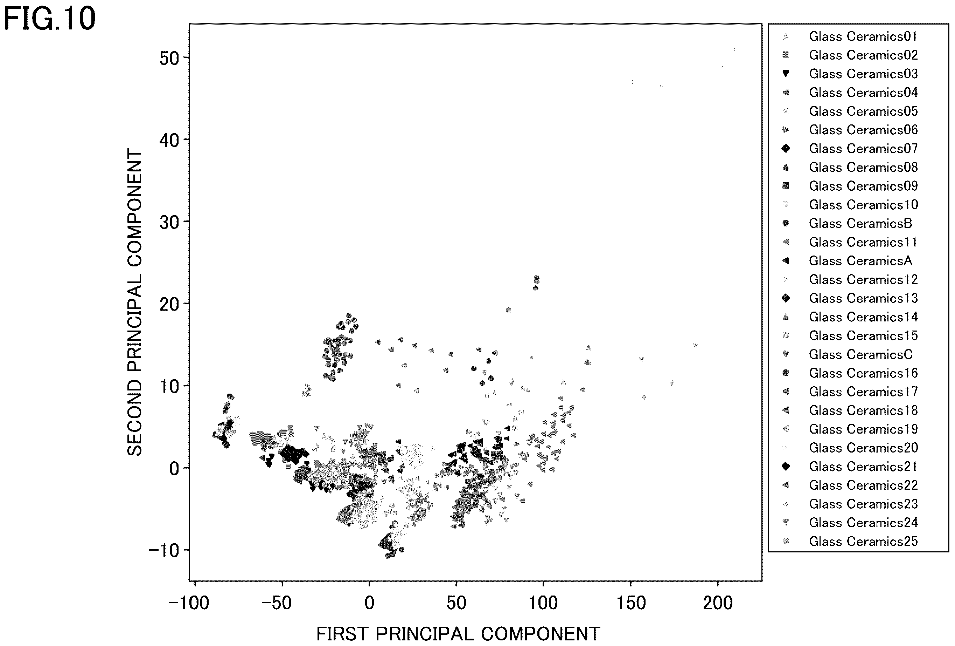

Next, the prediction device 10 and the learning device 20 performs dimensionality reduction of the features (vector). Figs. 9 and 10 are diagrams for explaining dimensionality reduction of a vector according to one embodiment of the present invention. As a result of extraction (vectorization) of features from the persistence diagram, one SEM image is converted into a vector having approximately 1,300 elements. Since 1,376 SEM images are used in total, the entire data is composed of a huge matrix of 1,300 × 1,376. If this data is used as it is, confirmation of the features by visualization or highly accurate prediction by machine learning cannot be achieved. Thus, dimensionality reduction of the features (vector) is performed by a principal component analysis.

Fig. 9 illustrates a cumulative contribution rate, where the vertical axis indicates a cumulative contribution rate and the horizontal axis indicates the number of principal components. As a result of the dimensionality reduction of the features (vector), it is confirmed that almost 100% of the original data can be explained with principal components including up to a second principal component.

In Fig. 10, the data is visualized using the first principal component (horizontal axis) and the second principal component (vertical axis). Each of prototypes or products forms a cluster, and is in a region that is slightly different from one another on the graph, thus it is confirmed that information specific to each material can be extracted. The feature visualization part 204 visualizes the features of the material by presenting a distribution of each material as in Fig. 10.

<<Machine learning>>

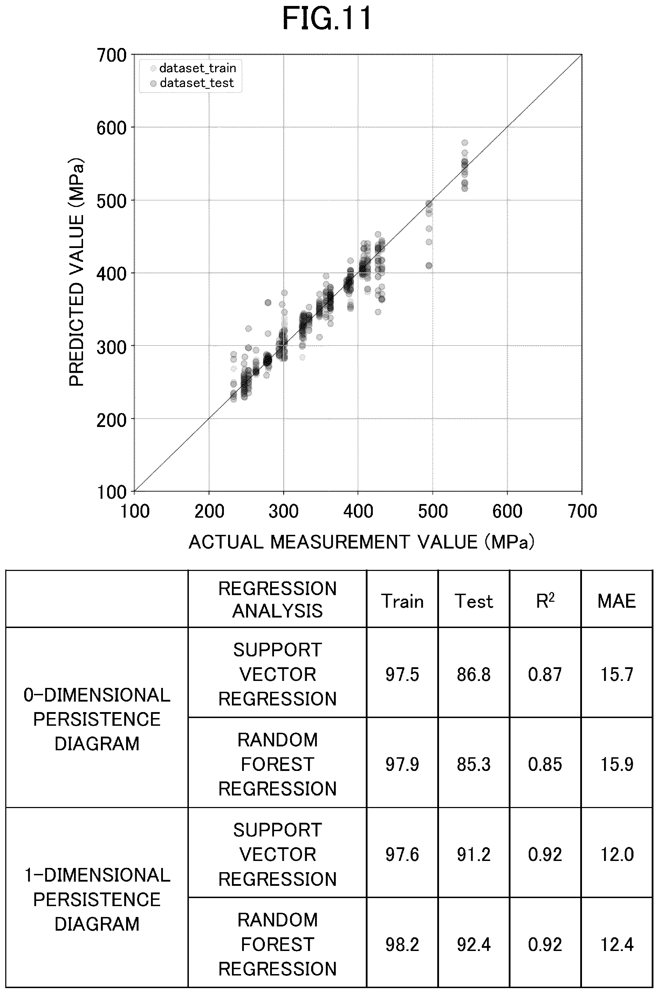

Next, the learning device 20 performs machine learning using the features (vectors). Fig. 11 is a diagram for explaining machine learning according to one embodiment of the present invention.

The data was condensed to 12 principal components through a principal component analysis (i.e., one SEM image can be expressed by a vector including 12 elements). By determining the 12 principal components as explanatory variables and a biaxial flexural strength (actual measurement value) of a glass-ceramic as an object variable, a regression analysis is performed by machine learning (e.g., support vector regression, random forest regression, etc.), and the accuracy presented in the bottom side of Fig. 11 is obtained ($R^2$ is around 0.9 in the test data). Note that, the hyperparameter of the machine learning model may be adjusted by an arbitrary optimizing algorithm. Examples thereof include grid search, random search, Bayesian optimization, and a genetic algorithm. The upper side of Fig. 11 presents actual measurement values (the actual measurement values (MPa) of the horizontal axis) of the biaxial flexural strength and predicted values (the predicted values (MPa) on the vertical axis) of the biaxial flexural strength for the training data (dataset_train) and the accuracy verification data (dataset_test).

<<Bayesian optimization>>

As described above, the learning device 20 can determine parameters ($\sigma$ (standard deviation), C, p of the equations (1) and (2)) used for extracting features of a material by Bayesian optimization. Moreover, the learning device 20 can determine the number of principal components, which is a parameter used for extracting features of a material, by Bayesian optimization. As the Bayesian optimization is performed approximately 50 times, a combination of optimal values is found.

Specifically, a predicted value of the characteristic value and a dispersion of the predicted value are calculated from the features of the material using the Gaussian process regression model to calculate an acquisition function. Optimum parameters are determined based on the acquisition function. As in the above manner, as a human merely produces and inputs training data, the learning device 20 can automatically learn through machine learning and can generate a trained model using the algorithm of the Bayesian optimization in combination.

<<Inverse analysis>>

An inverse analysis can be performed using the above persistence diagrams and the result of the principal component analysis.

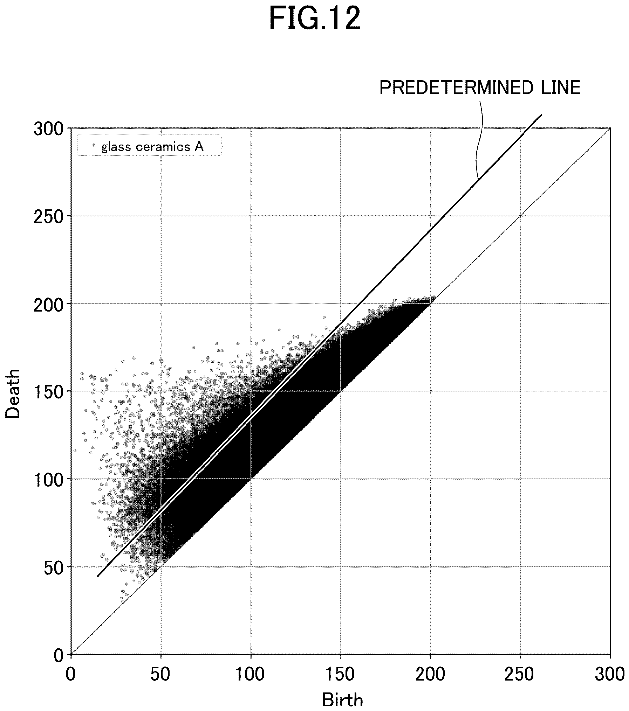

For example, as depicted in Fig. 12, the points having a longer life time (i.e., a period from birth to death) than a certain period on the persistence diagram (e.g., an upper left portion from the predetermined line of Fig. 12) are assumed to be important points on the image. Therefore, an important structure of crystals can be revealed by analyzing what kind of a structure of crystals corresponds to points having a longer life time than a certain period (e.g., an upper left portion with respect to the predetermined line of Fig. 12).

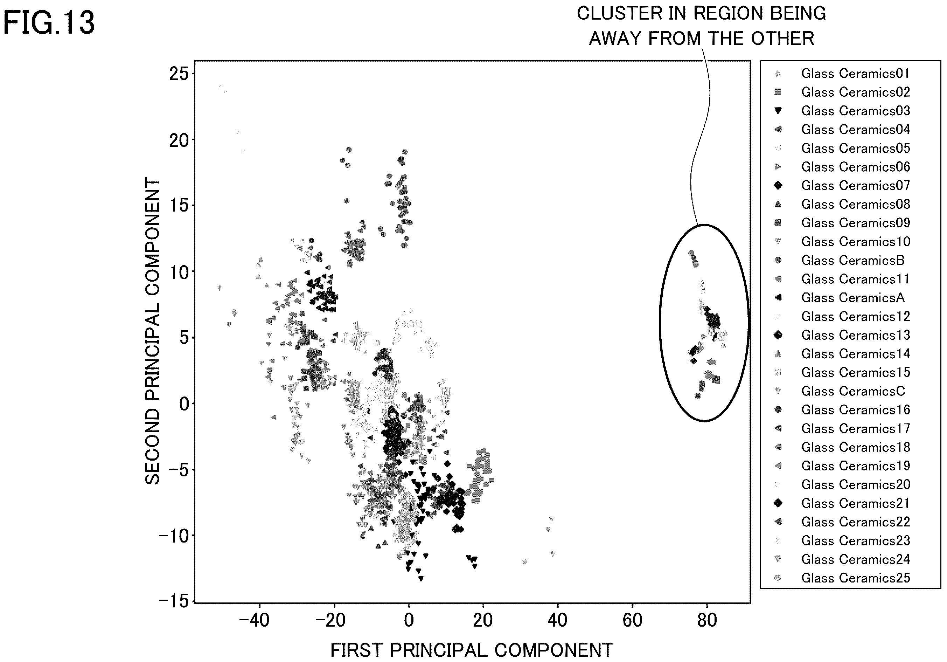

For example, as depicted in Fig. 13, what kind of a structure of crystals the points constituting the small cluster in a region being away from the other are derived from can be analyzed.

<Hardware configuration>

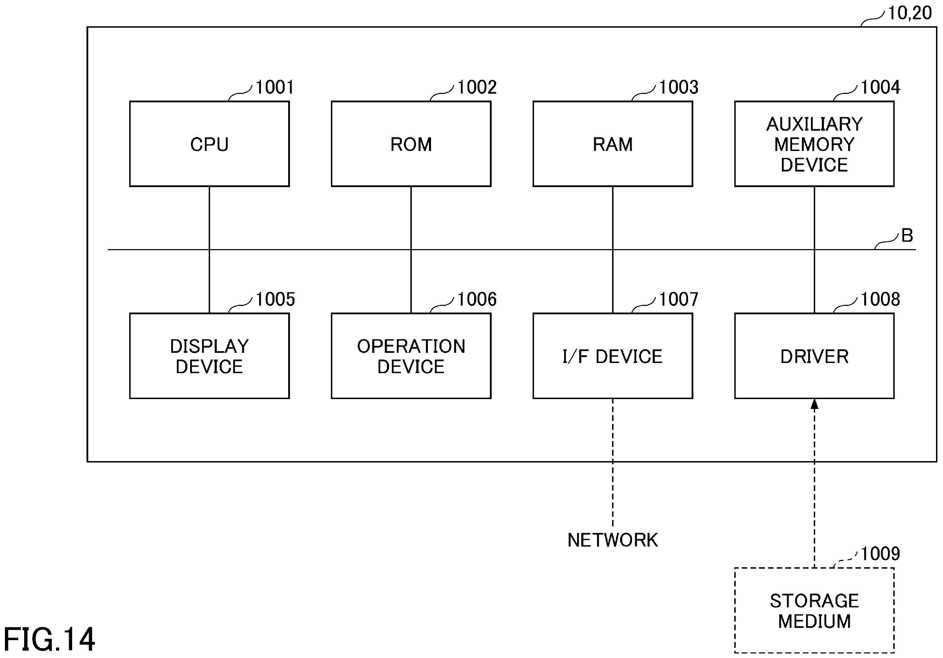

Fig. 14 is a diagram illustrating a hardware configuration of the prediction device 10 and the learning device 20 according to one embodiment of the present invention. The prediction device 10 and the learning device 20 include a central processing unit (CPU) 1001, a read-only memory (ROM) 1002, and a random access memory (RAM) 1003. The CPU 1001, the ROM 1002, and the RAM 1003 constitute a so-called computer. Moreover, the prediction device 10 and the learning device 20 may further include an auxiliary memory device 1004, a display device 1005, an operation device 1006, an interface (I/F) device 1007, and a driver 1008. Note that, the hardware components of the prediction device 10 and the learning device 20 are coupled to one another via a bus B.

The CPU 1001 is a computation device that executes various programs installed in the auxiliary memory device 1004. As the CPU 1001 executes the programs, the processes described in the present specification are performed.

The ROM 1002 is a non-volatile memory. The ROM 1002 functions as a main storage device that stores various programs, data, etc., necessary for the CPU 1001 to execute various programs installed in the auxiliary memory device 1004. Specifically, the ROM 1002 functions as a main storage device that stores boost programs, etc., such as a basic input/output system (BIOS), an extensible firmware interface (EFI), and the like.

The RAM 1003 is a volatile memory, such as a dynamic random-access memory (DRAM), a static random-access memory (SRAM), or the like. The RAM 1003 functions as a main storage device that provides a work space in which various programs installed in the auxiliary memory device 1004 are expanded when executed by the CPU 1001.

The auxiliary memory device 1004 is an auxiliary storage device that stores various programs and information used when the various programs are executed.

The display device 1005 is a display device that displays internal states and the like of the prediction device 10 and the learning device 20.

The operation device 1006 is an input device for a user who operates the prediction device 10 and the learning device 20 to input various instructions to the prediction device 10 and the learning device 20.

The I/F device 1007 is a communication device for connecting to the network to communicate with other devices.

The driver 1008 is a device for setting a storage medium 1009. The storage medium 1009 includes media for optically, electrically, or magnetically recording information, such as compact-disk (CD)-ROM, flexible disks, magneto-optical disks, and the like. The storage medium 1009 may include a semiconductor memory and the like that electrically record information, such as a ROM, a flash memory, and the like.

Note that, various programs to be installed in the auxiliary memory device 1004 are installed, for example, by setting a distributed storage medium 1009 in the driver 1008, and reading the various programs recorded on the storage medium 1009 by the driver 1008. Alternatively, various programs to be installed in the auxiliary memory device 1004 may be installed by downloading the programs from the network via the I/F device 1007.

Although the embodiments of the present invention have been described above in detail, the present invention is not limited to the above-described specific embodiments, and various modifications and variations are possible within the scope of the invention as claimed.

The present application claims priority to Japanese Patent Application No. 2022-055035, filed with the Japan Patent Office on March 30, 2022, the entire contents of which are incorporated in the present application by reference.

REFERENCE SIGNS LIST

10      prediction device
20      learning device
30      user

# EP 4 503 045 A1

| | |
|---|---|
| 101 | image acquisition part |
| 102 | feature extraction part |
| 103 | prediction part |
| 201 | training data acquisition part |
| 202 | feature extraction part |
| 203 | learning part |
| 204 | feature visualization part |
| 205 | optimization part |
| 1001 | CPU |
| 1002 | ROM |
| 1003 | RAM |
| 1004 | auxiliary memory device |
| 1005 | display device |
| 1006 | operation device |
| 1007 | I/F device |
| 1008 | driver |
| 1009 | storage medium |

**Claims**

1. A method comprising:

   acquiring an image of a material;
   performing a topological data analysis on the image of the material to extract features of the material; and
   predicting a characteristic value of the material from the features of the material.

2. A method comprising:

   acquiring an image of a material and an actual measurement value of a characteristic value of the material;
   performing a topological data analysis on the image of the material to extract features of the material; and
   producing a machine learning model with the features of the material and the actual measurement value of the characteristic value of the material to generate a trained model for predicting a characteristic value of the material from the features of the material.

3. The method according to claim 1 or 2,
   wherein the topological data analysis is persistent homology.

4. The method according to any one of claims 1 to 3,
   wherein the material is a ceramic, a glass-ceramic, a polymer material, a composite resin, a glass ionomer, or a metal.

5. The method according to any one of claims 1 to 4,
   wherein the characteristic value is a biaxial flexural strength.

6. The method according to any one of claims 1 to 5,
   wherein the image is an SEM image.

7. The method according to any one of claims 1 to 6, further comprising:
   performing dimensionality reduction of the features of the material.

8. The method according to any one of claims 1 to 7, further comprising:
   dividing the image and extracting the features of the material from the divided image.

9. The method according to claim 2, further comprising:
   visualizing the features of the material.

10. The method according to claim 2, further comprising:
    Determining parameters for extracting the features of the material through Bayesian optimization.

11. A program for causing a computer to execute processes comprising:

    acquiring an image of a material;
    performing a topological data analysis on the image of the material to extract features of the material; and
    predicting a characteristic value of the material from the features of the material.

12. A program for causing a computer to execute processes comprising:

    acquiring an image of a material and an actual measurement value of a characteristic value of the material;
    performing a topological data analysis on the image of the material to extract features of the material; and
    producing a machine learning model with the features of the material and the actual measurement value of the characteristic value of the material to generate a trained model for predicting a characteristic value of the material from the features of the material.

13. A device comprising:

    an acquisition part configured to acquire an image of a material;
    a feature extraction part configured to perform a topological data analysis on the image of the material to extract features of the material; and
    a prediction part configured to predict a characteristic value of the material from the features of the material.

14. A device comprising:

    an acquisition part configured to acquire an image of a material and an actual measurement value of a characteristic value of the material;
    a feature extraction part configured to perform a topological data analysis on the image of the material to extract features of the material; and
    a learning part configured to produce a machine learning model with the features of the material and an actual measurement value of a characteristic value of the material to generate a trained model for predicting a characteristic value of the material from the features of the material.

# FIG.1

~10

PREDICTION
DEVICE

~30

USER

~20

LEARNING
DEVICE

# FIG.2

~10

PREDICTION DEVICE

~101

IMAGE
ACQUISITION
PART

~102

FEATURE
EXTRACTION
PART

~103

PREDICTION
PART

# FIG.3

LEARNING DEVICE 20

TRAINING DATA ACQUISITION PART 201

FEATURE EXTRACTION PART 202

LEARNING PART 203

FEATURE VISUALIZATION PART 204

OPTIMIZATION PART 205

# FIG.4

```
                    ┌─────────────┐
                    │    START    │
                    └──────┬──────┘
                           │            S11
                           ▼
    ┌──────────────────────────────────────────────────┐
    │            ACQUIRE IMAGE OF MATERIAL              │
    └──────────────────────┬───────────────────────────┘
                           │            S12
                           ▼
    ┌──────────────────────────────────────────────────┐
    │                  DIVIDE IMAGE                     │
    └──────────────────────┬───────────────────────────┘
                           │            S13
                           ▼
    ┌──────────────────────────────────────────────────┐
    │  PERFORM TOPOLOGICAL DATA ANALYSIS ON IMAGE       │
    │  OF MATERIAL TO EXTRACT FEATURES OF MATERIAL      │
    └──────────────────────┬───────────────────────────┘
                           │            S14
                           ▼
    ┌──────────────────────────────────────────────────┐
    │        PERFORM DIMENSIONALITY REDUCTION           │
    │              OF FEATURES OF MATERIAL              │
    └──────────────────────┬───────────────────────────┘
                           │            S15
                           ▼
    ┌──────────────────────────────────────────────────┐
    │          PREDICT CHARACTERISTIC VALUE OF          │
    │        MATERIAL FROM FEATURES OF MATERIAL         │
    └──────────────────────┬───────────────────────────┘
                           │            S16
                           ▼
    ┌──────────────────────────────────────────────────┐
    │            PRESENT PREDICTED RESULT               │
    └──────────────────────┬───────────────────────────┘
                           │
                           ▼
                    ┌─────────────┐
                    │     END     │
                    └─────────────┘
```

# FIG.5

START

S21

ACQUIRE IMAGE OF MATERIAL AND ACTUAL
MEASUREMENT VALUE OF CHARACTERISTIC
VALUE OF MATERIAL

S22

DIVIDE IMAGE

S23

DETERMINE PARAMETERS

S24

PERFORM TOPOLOGICAL DATA ANALYSIS ON
IMAGE TO EXTRACT FEATURES OF MATERIAL

S25

PERFORM DIMENSIONALITY REDUCTION
OF FEATURES OF MATERIAL

S26

VISUALIZE FEATURES OF MATERIAL

S27

PERFORM MACHINE LEARNING TO
GENERATE TRAINED MODEL

END

FIG.6

<AFTER DIVISION>

<BEFORE DIVISION>

# FIG.7

<IMAGE>

⇒

<TEXT FORMAT>

| 12 | 23 | 34 | 45 | 56 | 67 | 78 | 89 | 91 |
|----|----|----|----|----|----|----|----|----|
| 23 | 34 | 45 | 56 | 67 | 78 | 89 | 91 | 12 |
| 34 | 45 | 56 | 67 | 78 | 89 | 91 | 12 | 23 |
| 45 | 56 | 67 | 78 | 89 | 91 | 12 | 23 | 34 |
| 56 | 67 | 78 | 89 | 91 | 12 | 23 | 34 | 45 |

⇒ STANDARDIZATION/
NORMALIZATION

EP 4 503 045 A1

# FIG.8

<PERSISTENCE DIAGRAM>

glass ceramics A

<TEXT FORMAT>

| 12 | 23 | 34 | 45 | 56 | 67 | 78 | 89 | 91 |
|----|----|----|----|----|----|----|----|----|
| 23 | 34 | 45 | 56 | 67 | 78 | 89 | 91 | 12 |
| 34 | 45 | 56 | 67 | 78 | 89 | 91 | 12 | 23 |
| 45 | 56 | 67 | 78 | 89 | 91 | 12 | 23 | 34 |
| 56 | 67 | 78 | 89 | 91 | 12 | 23 | 34 | 45 |

glass ceramics B

glass ceramics C

# FIG.9

EP 4 503 045 A1

FIG.10

# FIG.11

| | REGRESSION ANALYSIS | Train | Test | R² | MAE |
|---|---|---|---|---|---|
| 0-DIMENSIONAL PERSISTENCE DIAGRAM | SUPPORT VECTOR REGRESSION | 97.5 | 86.8 | 0.87 | 15.7 |
| | RANDOM FOREST REGRESSION | 97.9 | 85.3 | 0.85 | 15.9 |
| 1-DIMENSIONAL PERSISTENCE DIAGRAM | SUPPORT VECTOR REGRESSION | 97.6 | 91.2 | 0.92 | 12.0 |
| | RANDOM FOREST REGRESSION | 98.2 | 92.4 | 0.92 | 12.4 |

# FIG.12

# FIG.13

CLUSTER IN REGION BEING
AWAY FROM THE OTHER

| | |
|---|---|
| △ | Glass Ceramics01 |
| ▣ | Glass Ceramics02 |
| ▼ | Glass Ceramics03 |
| ◀ | Glass Ceramics04 |
| ◁ | Glass Ceramics05 |
| ▷ | Glass Ceramics06 |
| ◆ | Glass Ceramics07 |
| ▲ | Glass Ceramics08 |
| ▣ | Glass Ceramics09 |
| ▽ | Glass Ceramics10 |
| ● | Glass CeramicsB |
| ◀ | Glass Ceramics11 |
| ◀ | Glass CeramicsA |
| ▷ | Glass Ceramics12 |
| ◆ | Glass Ceramics13 |
| ▲ | Glass Ceramics14 |
| ▣ | Glass Ceramics15 |
| ▽ | Glass CeramicsC |
| ● | Glass Ceramics16 |
| ◀ | Glass Ceramics17 |
| ◀ | Glass Ceramics18 |
| ◁ | Glass Ceramics19 |
| ▷ | Glass Ceramics20 |
| ◆ | Glass Ceramics21 |
| ◀ | Glass Ceramics22 |
| ▲ | Glass Ceramics23 |
| ▼ | Glass Ceramics24 |
| ● | Glass Ceramics25 |

SECOND PRINCIPAL COMPONENT

FIRST PRINCIPAL COMPONENT

EP 4 503 045 A1

FIG.14

EP 4 503 045 A1

| INTERNATIONAL SEARCH REPORT | International application No. |
|---|---|
| | **PCT/JP2023/006045** |

**A. CLASSIFICATION OF SUBJECT MATTER**

***G16C 60/00***(2019.01)i; ***G01N 23/2251***(2018.01)i; ***G01N 33/00***(2006.01)i; ***G06T 7/00***(2017.01)i
FI: G16C60/00; G01N23/2251; G01N33/00 D; G06T7/00

According to International Patent Classification (IPC) or to both national classification and IPC

**B. FIELDS SEARCHED**

Minimum documentation searched (classification system followed by classification symbols)

G06Q10/00-99/00; G16C10/00-99/00; G01N23/2251; G01N33/00; G06T7/00

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | 平岡裕章ほか2名, マテリアルズインフォマティクス, 人工知能, 01 May 2019, vol. 34, no. 3, pp. 330-338, ISSN 2188-2266<br>p. 333, "5. Structural analysis of silica glass", pp. 336-337, "7. Machine learning for persistent diagrams", fig. 8, (Journal of Japanese Society for Artificial Intelligence), non-official translation (HIRAOKA, Yasuaki et al. Materials Informatics.) | 1-14 |
| A | JP 2019-179319 A (FUJITSU LTD) 17 October 2019 (2019-10-17)<br>entire text, all drawings | 1-14 |
| A | US 2009/0087070 A1 (SLABAUGH, Gregory G.) 02 April 2009 (2009-04-02)<br>entire text, all drawings | 1-14 |

☐ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **11 April 2023** | **25 April 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**
Information on patent family members

International application No.

**PCT/JP2023/006045**

| Patent document cited in search report | | | Publication date (day/month/year) | Patent family member(s) | Publication date (day/month/year) |
|---|---|---|---|---|---|
| JP | 2019-179319 | A | 17 October 2019 | (Family: none) | |
| US | 2009/0087070 | A1 | 02 April 2009 | (Family: none) | |

Form PCT/ISA/210 (patent family annex) (January 2015)

**EP 4 503 045 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2021111360 A
- JP 2022055035 A